# EUROPEAN PATENT APPLICATION

(11) **EP 2 560 110 A1**
(43) Date of publication of application: **20.02.2013**
(21) Application number: 12177400.4
(22) Date of filing: 28.05.2004
(51) Int. Cl.: G06F 17/30, G11B 3/00

(54) **Systems and methods utilizing natural language medical records**

(30) Priority: 29.05.2003 US 447290
(62) Divisional of application: 04753663.6
(71) Applicant: Dictaphone Corporation, Stratford, CT 06614-2566 (US)
(72) Inventor: Boone, Keith W, Massachusetts, MA Massachusetts 02368 (US); Carus, Alwin B, Massachusetts, MA Massachusetts 01890 (US); Deplonty, Thomas J, Massachusetts, MA Massachusetts MA 02176 (US); Hopkins, Jeffrey G, Rhose Island, RI Rhode Island RI 02865 (US); Ogrinc, Harry J, Rhode Island, MA Massachusetts MA 02052 (US); Reggie, Susan, New York, NY New York NY 10601 (US); Titemore, Robert G, Massachusetts, MA Massachusetts MA 02420 (US)
(74) Representative: Barton, Russell Glen

(57) **Abstract**

The invention involves systems and methods for generating, manipulating, summarizing, storing, reusing, and searching electronic medical records. Structured input (20) of medical information by medical personnel based on templates may optionally be used to facilitate analysis of records, while allowing less restrictive text input than systems of the prior art. Data extraction (25) of relevant medical data from the input text may optionally be facilitated by the structured format of the medical records. Extracted medical data is optionally validated and linked or associated with the text from which it was extracted. The extracted medical data is normalized to allow easier searching (30) than available in systems of the prior art. Medical and document metadata is incorporated into the extracted medical data. Particularly pertinent medical information may be extracted and summarized from a patient's medical history for use by a medical professional at the point of care.

## Description

### BACKGROUND OF THE INVENTION

Hospitals, medical clinics, medical offices, and other sources of medical care typically keep records for their patients. These records include a variety of information such as physician and nursing notes regarding a patient's complaints and symptoms, diagnoses, treatments and procedures administered, allergies, medicines the patient has been taking, and medicines that are newly prescribed. Medical records allow physicians who treat a patient in the future to gain background regarding the patient's condition, allow hospitals to gauge the process and quality of care, and are frequently used for billing purposes, outcomes analysis and decision support. A great deal of information is generated for each patient. In hospital or clinical environments, where numerous patients are treated, the volume of information generated for all patients can become truly enormous, thus creating an ever-present need for more efficient ways of storing, summarizing, reusing, and retrieving the information.

One of the ways that the healthcare industry has developed to manage healthcare information involves the standardization of nomenclature for diagnoses, treatments, medical procedures, medications, and other medical services. Many systems of standardization exist. One system is the International Classification of Diseases (ICD-9-CM, which indicates the 9th revision and a US clinical modification of ICD-9, published by the World Health Organization), published by the US Government. The International Classification of Diseases is a classification structure that provides rules for assigning numeric codes that specify diseases, injuries, the causes of these, medical findings, and other factors affecting patient care, as well as codes for surgical, diagnostic, and therapeutic procedures. Other systems of medical classification include the Current Procedural Terminology (CPT), published by the American Medical Association (AMA), which provides classification codes for surgical, radiological, diagnostic, and therapeutic services, as well as codes for services provided in various medical specialties and laboratory procedures. Another classification system is the Systemized Nomenclature of Medicine Clinical Terms (SNOMED CT), published by the College of American Pathologists (CAP), which provides detailed and specific classification codes for clinical information and reference terminology and is cross-referenced to the ICD-9-CM.

Notwithstanding the variety of options available for standardization of medical records, physicians and other healthcare providers often fail to use classification codes in creating medical records because classification usually involves significant effort and is not worth the physicians' time. However, healthcare providers are often required to provide standardized medical reports in order to recover expenses from insurance providers. Furthermore, the medical community can benefit from standardized medical records for such purposes as statistical analyses of disease and epidemic containment. Thus healthcare providers often employ coding specialists, who review patients' medical records, extract information regarding medical services provided and diagnoses made, manually look up the classification codes for those services, and annotate the medical record with the codes corresponding to the services provided. Coded summaries of the encounters may be provided to insurance companies for billing purposes, or they may be made part of a medical record, in part or in their entirety, providing a shorthand notation for various symptoms, diagnoses, treatments, prescribed drugs, etc.

An option for increasing the reliability, consistency, and efficiency of coding is to add automation to the process. Automated coding engines are text processors for parsing free medical text, such as that written or dictated by a physician while diagnosing or treating a patient, and translating it into a system of medical codes for any number of purposes. Coding engines sort through input medical text, rearranging and annotating the material, searching for a reasonable match of the input medical text to a database of predetermined medical descriptions corresponding to particular classification codes.

A persistent goal of many medical facilities is to move to a completely electronic medical record (EMR). An EMR would replace a patient's paper medical record and provide an electronic record that is easily accessible, searchable, editable, and potentially reusable. An EMR may be based on any of the existing classification schemes described above, or it may be based on a unique, customized scheme. Notwithstanding the desirability of EMRs, the transition is often too drastic, requiring a completely new approach to creating medical records. The physicians and other medical personnel who must create the medical records are often hesitant to take the extra time involved to learn to create records within EMRs. For example, many physicians prefer to dictate their medical reports, but options presently available for generating EMRs require manual data entry. Data entry can be difficult and time-consuming, often requiring physicians to answer specific questions (many of which are not relevant), rather than allowing the free-form dictation that physicians are used to. Such data entry requirements make inefficient use of a physician's limited time. EMR vendors generally require physicians to train for 10-30 hours to learn to use the system. Consequently, physician acceptance is low. Many physicians do not use the system to generate reports while many physicians only use the system to view reports. This often results in incomplete information in the database.

There is thus a need in the art for a system that is simple and easy to learn and use, with a convenient user interface, for generating, editing, storing, searching, and potentially reusing EMRs. There is a particular need in the art for an EMR system that does not require physicians and other medical professionals to substantially change the way they currently generate medical records.

There is a further need in the art for a system that provides file handling and workflow capabilities for the generation of EMRs from various forms of medical record data, for handling automated speech recognition for reducing voice data to text, and for extracting essential information from medical data.

Another approach to medical documentation is the Computerized Data Repository (CDR). A CDR is a document-centric repository of medical reports. Although searching CDRs in an electronic database is easier than searching paper medical records, searching in prior art systems using CDRs is difficult because different physicians often use different words for the same medical terms. Thus, a searcher who wishes to find a group of medical records that involve a particular medical term would have to know and use all of the variants of that term in order to ensure a complete search. There is thus a further need in the art for a system to facilitate searching CDRs.

"EMR systems" may refer to those medical records that are created via tedious, highly structured data entry (as opposed to via dictation). In such systems, data is typically normalized. It is desirable to provide a system that it takes advantage of the benefits of an EMR system, namely, normalization for the most important medical data, but does not force providers to change the way they generate medical records.

### SUMMARY OF THE INVENTION

In light of the above identified deficiencies of the prior art, it is thus an object of the present invention to provide systems and methods that allow generation of a summarized electronic medical record without requiring medical personnel to substantially change their practices in creating medical records.

In a first aspect, the present invention includes system for managing information. The system may include a means for capturing text from a document source, a means for determining the structure of the captured text, a means for extracting elements of data from the captured text, a means for categorizing the extracted elements of data, a means for normalizing the extracted elements of data, a means for adding metadata relating to the captured text, a means for validating the extracted elements of data and the metadata, wherein the means for validating further includes validating at least one of the normalized extracted elements of data and the categorized extracted elements of data, a storage means for storing an electronic document including the categorized, normalized and validated extracted elements of data and metadata in a storage means, wherein the categorized, normalized and validated extracted elements of data and metadata are stored in association with the captured text; and a means for retrieving the electronic document from the storage means.

In some embodiments the system includes a means for displaying the retrieved electronic records, a means for displaying the metadata, and a means for displaying the captured text. The system may also include a means for determining the structure of the format of the captured text. The means for determining may include determining the structure of the content of the captured text, wherein the content of the captured includes one of terms, words and phrases and a means for determining the overall classification of the content of the captured text.

In some embodiments, the system may include a means for reusing the extracted elements of data, the metadata or the captured text. The system may also include a means for tracking which sections of the captured text have been completed.

In some embodiments the document source originates from an archive of legacy documents and may include at least one new document created from a predetermined document template. The system may also include means for entering text in the new document created from the predetermined document template, wherein the means for entering text in the new document created from a predetermined document template may include one of a keyboard attached to a computer, a microphone attached to a computer, a telephone, or a PDA.

In some embodiments, the storage means for storing the electronic document is a computer hard drive, the means for extracting elements of data from the text is a data extraction engine and the means for validating extracted elements of data is a software program with access to the storage means for storing an electronic record, wherein the means for retrieving is accomplished by reference to at least one of the extracted elements of data, the metadata or the captured text. The means for retrieving an electronic document from the storage means may include a software program with access to the storage means for storing an electronic record, wherein information about the location of the extracted elements of data within the captured text is used to retrieve a section of the electronic document corresponding to the location of the extracted elements of data.

In some embodiments the means for adding metadata may include a software program with access to the storage means for storing an electronic document, the means for categorizing includes canonicalizing the extracted of elements of data. The means for categorizing may include classifying headings contained in the extracted elements of data.

In a second aspect, the present invention includes a method for managing information. The method may include capturing text from a document source, determining the structure of the captured text, extracting elements of data from the captured text, categorizing the extracted elements of data, normalizing the extracted elements of data, adding metadata relating to the captured text, validating the extracted elements of data and the metadata, wherein validating further includes validating at least one of the normalized extracted elements of data and the categorized extracted elements of data, storing an electronic document including the categorized, normalized and validated extracted elements of data and metadata in a storage means, wherein the categorized, normalized and validated extracted elements of data and metadata are stored in association with the captured text, and retrieving the electronic document from the storage means.

In some embodiments the method may include displaying the retrieved electronic records, the metadata or the captured text. The method may also include determining the structure of the format of the captured text, determining the structure of the content of the captured text, wherein the content of the captured may include one of terms, words and phrases, and determining the overall classification of the content of the captured text.

In some embodiments the method may include reusing the extracted elements of data, the metadata or captured text. The method may also include tracking which sections of the captured text have been completed.

In some embodiments the document source originates from an archive of legacy documents, wherein the document source includes at least one new document created from a predetermined document template. The method may also include entering text in the new document created from the predetermined document template, wherein step of entering text in the new document created from a predetermined document template includes one of a keyboard attached to a computer, a microphone attached to a computer, a telephone, or a PDA.

In some embodiments the method includes storing the electronic document is storing the electronic document on a computer hard drive. The method may also include extracting elements of data from the text using a data extraction engine.

In some embodiment the method may include validating extracted elements of data using a software program with access to the storage means for storing an electronic record. The method may also include retrieving by reference to at least one of the extracted elements of data, the metadata or the captured text, wherein the retrieving an electronic document includes using is a software program with access to the storage means for storing an electronic record. In addition, the method may include information about the location of the extracted elements of data within the captured text is used to retrieve a section of the electronic document corresponding to the location of the extracted elements of data.

In some embodiments the method may include adding metadata using a software program with access to the storage means. The categorizing step may include using canonicalizing the extracted of elements of data and, wherein the step of categorizing may also include classifying headings contained in the extracted elements of data.

In a third aspect, the present invention may include a method for generating, editing, storing, and searching electronic medical records. The method may include entering medical information text in a predetermined medical record template, wherein text pertinent to a section of the template is entered in that section as natural language; storing a completed medical record; extracting elements of medical data from the text, wherein information about which section of the template the text was entered is used to extract elements of medical data from the text; validating extracted data; normalizing the extracted elements of medical data; storing an electronic medical record as normalized extracted elements of medical data in association with the text from which they were extracted; and retrieving electronic medical records from the storage means by natural language searching.

In one embodiment, the step of entering medical information text is performed using a microphone attached to a computer. In another embodiment, the step of entering medical information is performed using a telephone. In another embodiment, the step of entering medical information is performed using a PDA. In another embodiment, the data may originate from archives or other document repositories in the form of electronic texts. In another embodiment, the steps of storing a completed medical record and of storing an electronic medical record are performed on computer hard drives. In another embodiment, the steps of storing a completed medical record and of storing an electronic medical record are performed on database servers. In another embodiment, the step of extracting elements of medical data from the text is performed using a NLP and ML data extraction engine. In another embodiment, the step of validating extracted data is performed using a web browser software program with access to the stored electronic medical record. In another embodiment, the step of normalizing the extracted elements of medical data is performed using a software program running on a computer.

In another embodiment, the step of retrieving electronic medical records is performed using a web browser software program with access to the stored electronic medical record. In another embodiment, the method further comprises the step of creating a medical record template. In another embodiment, the method further comprises the step of providing a patient history summary. In still another embodiment the present invention includes the step of adding medical metadata. In other embodiments the additional medical metadata is stored on a computer hard drive or stored on a database server. In still other embodiments the step of adding medical metadata is performed using a software program running on a computer. In yet another embodiment the step of adding medical metadata is performed using a web browser with access to the stored electronic medical record.

In another aspect, the present invention may optionally include providing structure for a medical record, such as a medical record template with predetermined sections, wherein medical personnel enter relevant medical data in the different sections. This provides the additional benefit of making medical documentation more consistent across an institution. An advantage of the present invention is that it allows patient encounters to be summarized using a minimal set of clinical data extracted from dictation or other data entry.

Structured input can make for more complete and consistent medical records because it organizes the medical data and ensures that the medical professional entering the data considers each of the issues raised by each of the predetermined sections. Second, structured input simplifies the task of extracting salient data from the predetermined sections because some information about the data is known *a priori,* namely, what the information in each section relates to. Third, the structure of the resulting record facilitates searching records because, for example, searches across all records for a particular illness, treatment, medication, or procedure may be focused by limiting the search to a particular section of the records. Finally, information within a single medical record may be extracted, organized, summarized, reused, and searched in any number of ways based on the sectional organization of the record. Thus a "snapshot" of a patient's medical history may easily be generated, which can increase the efficiency with which medical personnel provide medical services, because they do not have to conduct a detailed review of a potentially long medical history. The snapshot view can provide the needed information more quickly and easily.

The invention may optionally include medical records that are organized according to a predetermined template. The text that is entered by a medical professional in each section may be stored in a database in association with that section, and may be searchable, either as an entire medical record, or section by section.

In one aspect, the invention may include a "scorecard" feature in which a computer program keeps track of which sections of the predetermined medical template have been completed, and displays this information in a graphical environment. In one embodiment, the invention may include a graphical user interface in which one portion of the screen shows text that has been entered by a medical professional, either through dictation and speech recognition, or through direct entry of text. Another portion of the screen may provide feedback to the medical professional to indicate the degree of progress made in completing the medical record. The scorecard feature may provide feedback on the degree to which the report matches some predetermined normative guidelines, as set forth by a particular site. For example, the scorecard might indicate which sections of a medical record template are mandatory for a particular site, and which of the mandatory sections have been completed.

Also associated with the medical records are pieces of medical data organized by data elements. For example, the elements of medical data may include such information as diagnoses or complaints, medications prescribed, patient allergies, medical procedures performed, results of laboratory tests, and any vaccinations or immunizations. Alternatively, the elements of medical data may include more or fewer basic pieces of information. These elements of medical data may be linked to the medical records, and can be searched. For example, a search for a particular type of drug may return the medical records of all patients who have been prescribed that drug, because that drug was one of the elements extracted from those records.

The above advantages and features are of representative embodiments only, and are presented only to assist in understanding the invention. It should be understood that they are not to be considered limitations on the invention as defined by the claims, or limitations on equivalents to the claims. Additional features and advantages of the invention will become apparent from the drawings, the following description, and the claims.

### BRIEF DESCRIPTION OF THE DRAWINGS

While the specification concludes with claims particularly pointing out and distinctly claiming the present invention, it is believed the same will be better understood from the following description taken in conjunction with the accompanying drawings, which illustrate, in a non-limiting fashion, the best mode presently contemplated for carrying out the present invention, and in which like reference numerals designate like parts throughout the figures, wherein:
FIG. 1 is an overview of the functionality of an embodiment of the invention;
FIG. 2 is a flow diagram showing how default templates for medical documents may be created and/or customized for use at a particular medical facility;
FIG. 3 is a flow diagram showing how a medical care provider can dictate and edit a medical record using an embodiment of the invention;
FIG. 4 is a flow diagram showing other methods by which a document may be entered into a system, by telephone following template outline, or on a PDA via a template established by the medical facility;
FIG. 5 is a flow diagram showing transcription workflow;
FIG. 6 is a flow diagram showing the steps in an embodiment of the data extraction and storage processes;
FIG. 7 is a flow diagram showing the steps in a database query using an embodiment of the data storage method of the invention; and
FIG. 8 is a flow diagram showing the steps in an embodiment in which previously stored documents are imported.

### DETAILED DESCRIPTION

The invention includes systems and methods for inputting, processing, and storing documents. In one embodiment, the invention includes systems and methods for inputting, processing, and storing medical records. While the following description is in terms of medical records, the concepts underlying the invention are applicable to inputting, processing, and storage of any sort of document, and can easily be adapted to documents other than medical records by those of ordinary skill in the art.

The invention as described in detail below provides many benefits to those involved in the production and use of medical records. For example, a physician can review the extracted elements of medical data, then easily obtain details about any element by retrieving the text in the EMR that served as the basis for the extraction of that element. The present invention may also allow a longitudinal view of the data over time. For example, the invention may be used to trace particular elements through a patient's medical history, thus allowing a quick review of laboratory results, medications, allergies, or other basic medical information over time. The invention especially provides benefits to database searching operations. For example, it allows a searcher to retrieve the medical records of all patients with a particular ailment, undergoing a particular treatment, or taking a particular medication. It allows a searcher to find all instances within an individual patient's record of an ailment, treatment, or medication of interest. Finally, it allows medical data to be easily shared between remote locations.

The invention can be comprised of distinct physical modules, which may be used all together, in a single system, or which can be used individually or in any combination with each other, or with other (e.g., third party) modules. Particularly, the physical modules can interface very effectively with presently existing dictation and transcription workflow systems that are already deployed in many medical facilities. In one embodiment, the modules may comprise a voice (or other data form) input system, a "physician workstation" for entering and editing medical data, a data extraction and normalization component, and a data viewing and searching component. Data extraction can be by any means known to those skilled in the art. For example, data extraction may proceed according to the natural language processing (NLP) and machine learning (ML) methods described in co-pending patent application serial no. 60/436,456, incorporated herein by reference in its entirety for all purposes. Extracted data may be verified by the physician or medical professional who entered the data from which the extracted data was extracted, or by a clinical data specialist.

One benefit of the present invention is that it allows the efficient use of previously stored data. After a medical professional has seen a patient once, on subsequent visits, the physician may access parts of any previous medical reports for the patient, and optionally insert those parts into the current medical report. This process is very efficient, allowing physicians to re-use data that is still relevant, without requiring them to enter the data again. This feature is particularly useful for discharge summaries after inpatient visits to a hospital or rehabilitation facility by allowing all procedures, treatments, diagnoses, and prescriptions provided to patients during their visits to be simply and easily summarized by amalgamating the reports generated during their visits. Thus a medical professional need only validate or verify the information that was pre-populated in the medical reports, and possibly dictate or otherwise add an addendum. A further benefit of this feature is that it expedites the creation of discharge summaries, which in turn allows for faster billing because insurers require the discharge summary before reimbursement.

Another benefit of the present invention is its improved EMR searching capability. The system of the invention can normalize text in medical reports to conform to a predetermined standard. For example, data extracted by NLP and ML may be stored as elements linked to the original text. The extracted elements can be normalized by associating a standard, predetermined set of medical terms corresponding to the extracted elements. This normalization facilitates searching because only a single term need be used in order to automatically retrieve all EMRs in which a medical professional used a word or phrase equivalent to that term. The system can further provide improved EMR searching capabilities by normalizing search queries, thus allowing any equivalent to a recognized medical term to be entered as a search query and to be recognized as equivalent to that term. Query results can simply be reported in the user interface of a computer program, printed out in a report, or exported in various formats, e.g., text, HL7, or XML.

One benefit of the present invention is its workflow solution. Medical professionals can begin the workflow by dictating medical data over the phone, on a mobile recorder, into a microphone on a computer, or by any other means of providing descriptive (natural language) medical information in electronic form. The natural language medical information may be entered according to some predetermined format or template, and may be edited by the medical professional entering the data. The natural language medical information may be transmitted, possibly to a remote location, to be subjected to speech recognition, if it is in the form of speech, or may simply be transcribed by a transcriptionist. The recognized text may then be transmitted back to the physician, who can verify the information, make any corrections, and sign off on the resulting report. Next, the text may be subjected to data extraction, possibly using a data extraction system such as the NLP and ML methods set forth in co-pending patent application serial no. 60/436,456. The extracted data may be optionally sent to a physician, nurse, or a clinical data specialist for validation. The validated data may be stored in a database in association with the text from which it was extracted along with associated patient and document metadata. The database may allow the data to be viewed, searched, or exported to other systems.

In a preferred embodiment of the invention, the normalization step is performed before the validation step. NLP and ML extracted elements can be normalized by associating a standard, predetermined set of medical terms corresponding to the extracted elements. Next, the normalized NLP and ML extracted elements can be optionally verified by a physician or clinical data specialist, who confirms that the normalized NLP and ML extracted elements are supported by the text from which they were extracted.

A further benefit provided by the present invention is that it allows a list of current allergies, problems, and medications to be maintained for each patient. Thus, for each visit with a medical professional, the system may provide the current list of allergies, problems, and medications, and during a preliminary examination, the medical professional may inquire of the patient whether anything has changed, whether they are still taking any recorded medications, whether any recorded problems have become better or worse, or any other material changes have occurred. The medical professional may then quickly and easily update the list, and the update may be reflected in the report generated for the current visit.

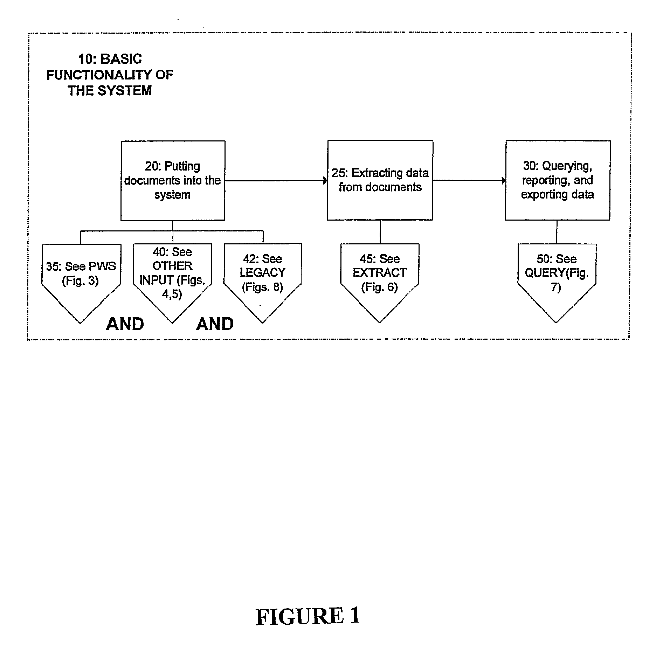

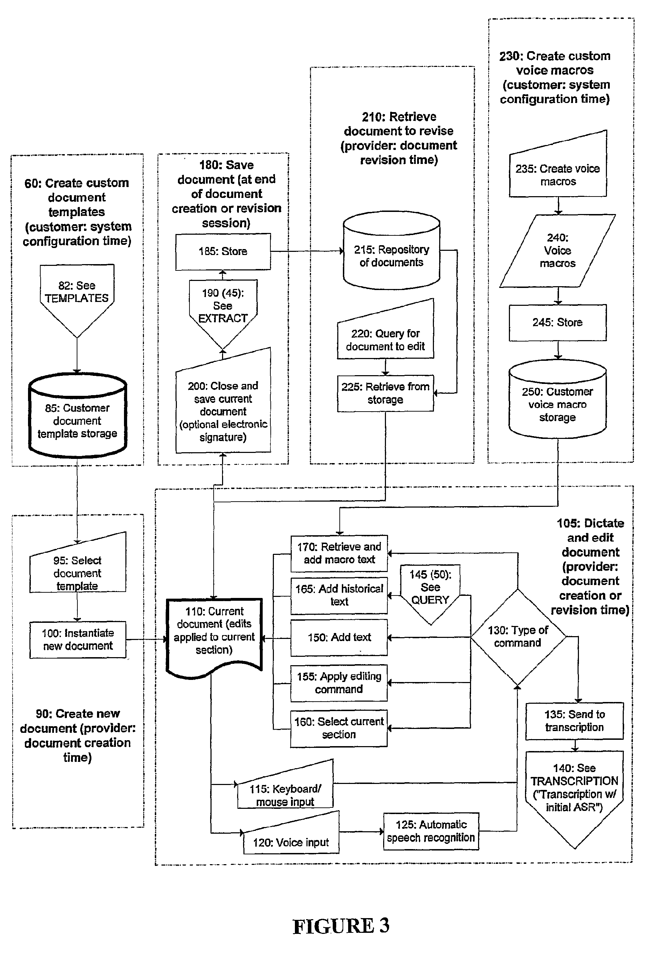

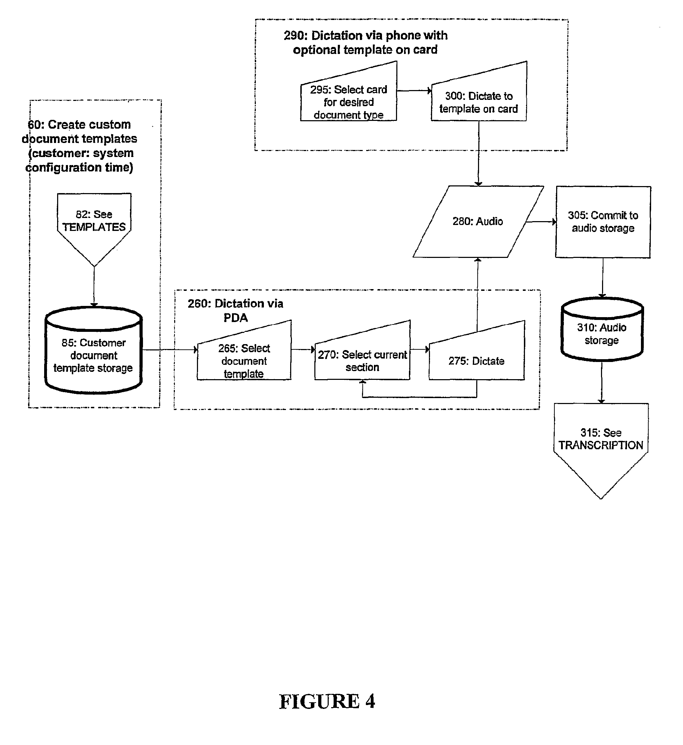

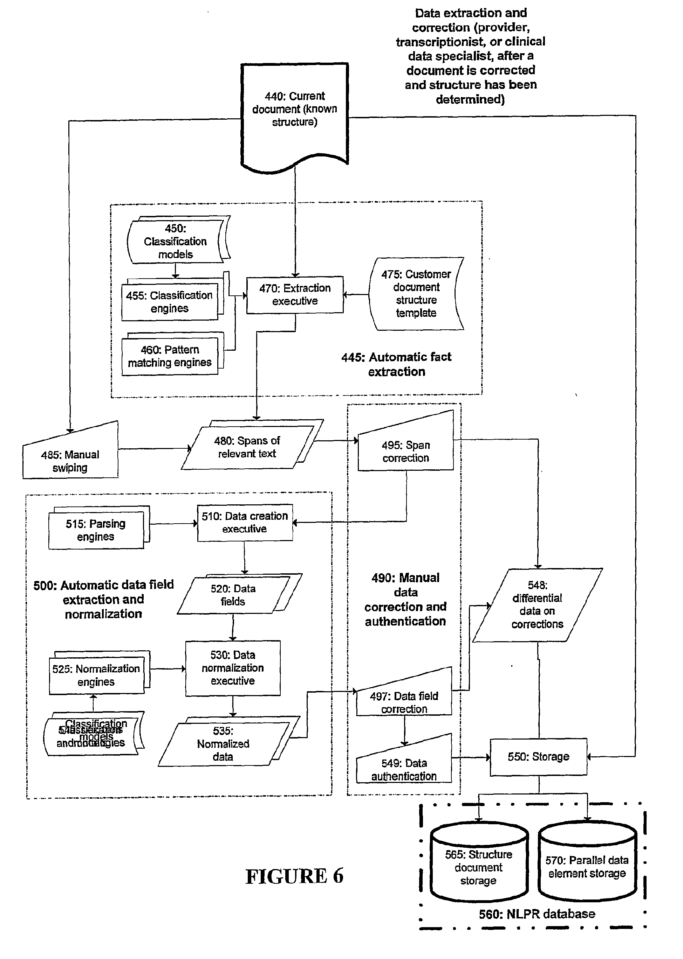

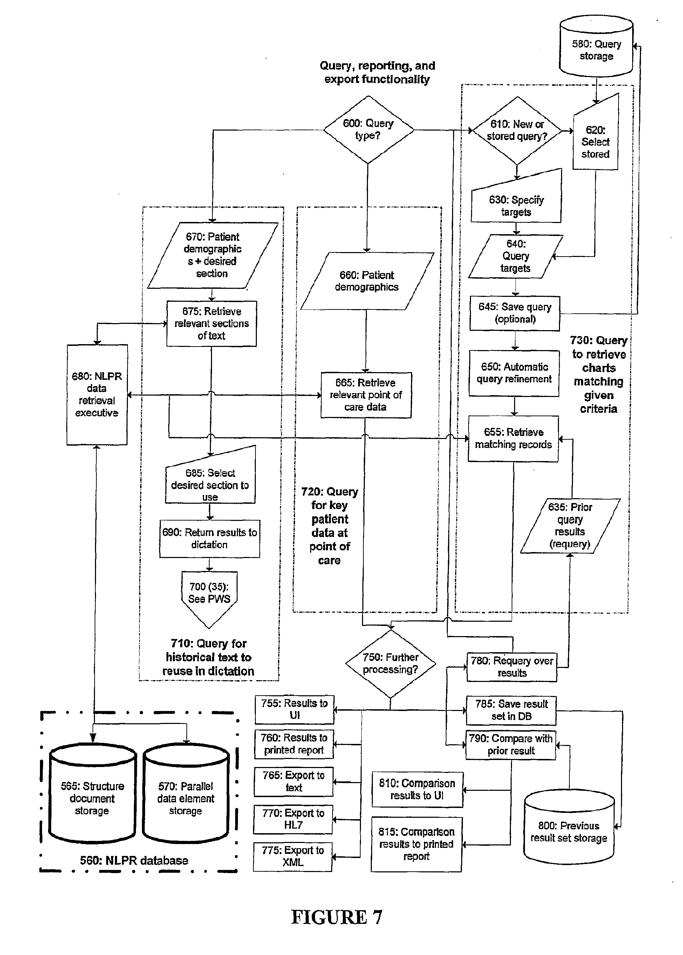

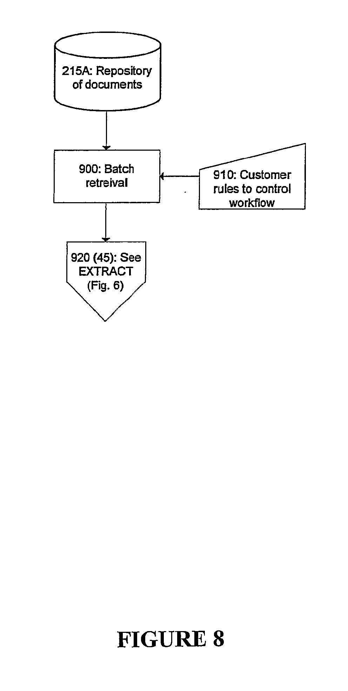

Turning now to FIG. 1, there is shown an overview of the main components of the systems and methods of the invention. Box 20 represents the systems and methods for inputting documents, which may be by use of a physician workstation (PWS) 35, as detailed in FIG. 3, by other input methods 40, two of which are detailed in FIG. 4, or which can be retrieved via a batch interface from a repository of previously stored documents 42, as detailed in FIG. 8. Box 25 represents the systems and methods for extracting data from inputted documents, which may be accomplished as detailed in FIG. 6. Box 30 represents the systems and methods for querying, reporting, and extracting data, which may be accomplished as detailed in FIG. 7.

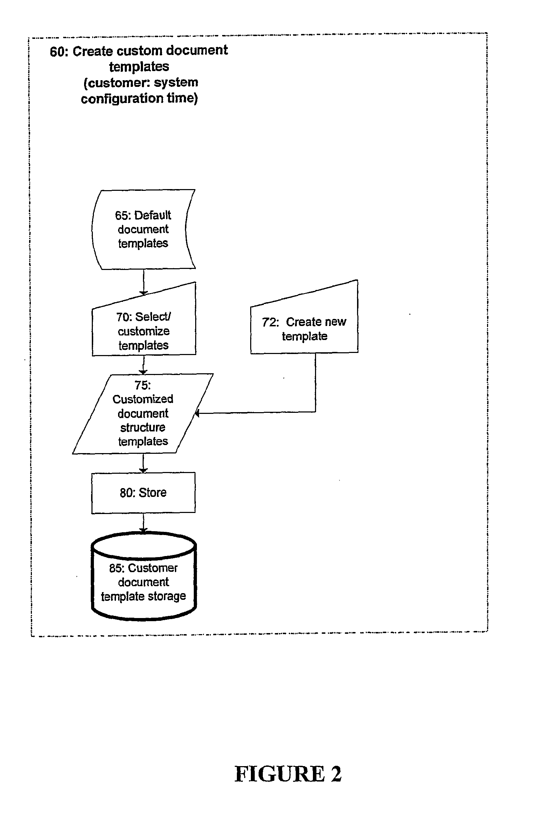

FIG. 2 is a flow diagram showing how templates for medical documents may be created and/or customized for use at a particular medical facility. Templates are an optional aspect of the invention; the invention should not be considered to require templates. Default shell templates may be provided in step 65. A customer, a medical facility in the case of medical records, may select a template based on a particular work type in step 70. The customer may then customize that template by adding or editing the wording of headings, or by adding sections or subsections to the document, for example, thus creating a customized document structure template 75. Alternately, the customer may create a template without using a default shell template in step 72. The customized document structure templates may then be stored 80 in a database 85 for use by medical personnel to complete during the course of their medical duties.

FIG. 3 is a flow diagram showing how a medical care provider can dictate and edit a medical record using an embodiment of the invention. This process may be understood in terms of distinct segments representing different sub-processes within the process. Sub-process 60 represents the storage of document templates 82 and 85, as described in the preceding paragraph and in FIG. 2. This sub-process is an optional process within the invention. The document templates may be the starting point for any document which a medical care provider may dictate or otherwise create.

Sub-process 90 allows medical care providers to create new documents. A medical care provider may select a document template 95 from the document template repository 85, which instantiates a new document 100. Document templates are an optional aspect of the invention.

Sub-process 230 allows a particular medical facility to optionally create custom voice macros. Custom voice macros may include boilerplate or specialized text that may be inserted into a document simply by speaking the call words for the voice macros. In step 235, a medical facility may create voice macros 240, which may be stored in step 245 in a persistent database 250 of customized voice macros.

Sub-process 210 allows medical care providers to retrieve documents they have been working on from a repository of documents 215. The providers may query 220 the database 215 for documents they have been working on in order to edit them. Any documents that satisfy the query 220 may be retrieved 225 from the database 215.

Sub-process 105 is the dictation and editing process; the current document being dictated is item 110. A physician or other dictator may provide input during this process, either through the computer keyboard or mouse, 115, or by speaking into a microphone at the workstation, 120, or by any other suitable means for providing input. If the input is dictated, the spoken words may be interpreted by computer voice recognition 125.

Spoken words or keyboard or mouse events may be designated as editing commands in a step that determines whether a command has been given, and parses the command 130. Editing commands may include selecting the current section 160 if dictation is done section by section within a document template 95. Other editing commands may specify that the cursor is to be moved to a particular place, or that a particular segment of text should be moved or deleted, or any number of other standard text editing commands 155. An editing command may direct that text is added to the current section 150. Another editing command may invoke the specifications of a query that can be done (see Fig. 7 and accompanying text below) to retrieve historical archived text, and allow that text to be inserted into the current document 165. Another command may invoke a voice macro, customized and stored in a macro database 250, such as created in sub-process 230, discussed above.

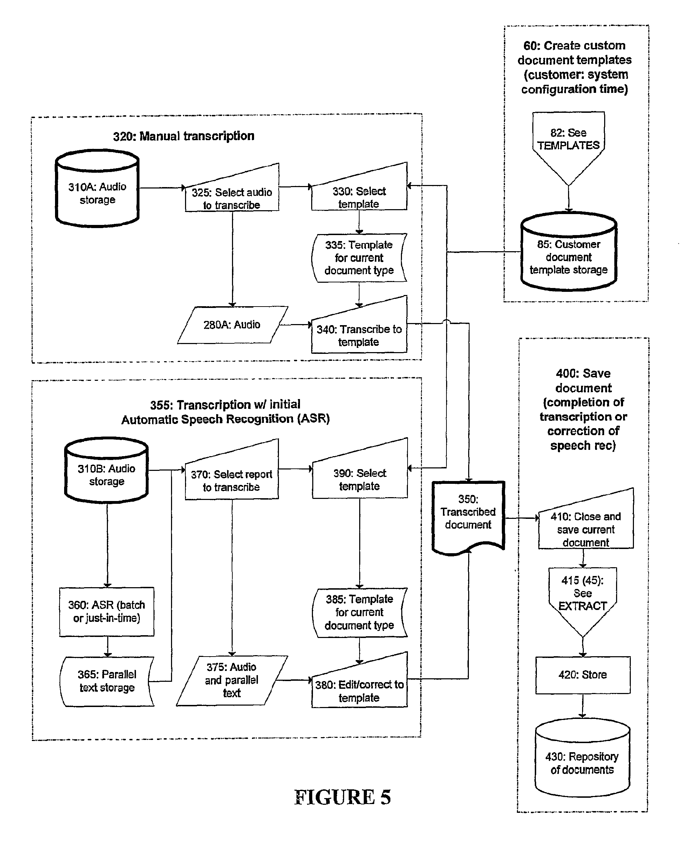

When a physician or other dictator is finished dictating and editing the current document, the document may be sent to a transcriptionist 135, where the editing of the document is completed 140 as shown in Fig. 5 and described in the accompanying text below.

Sub-process 180 details the steps in saving a document that has been entered and edited. The document is closed and saved in step 200, and the physician has the option of digitally "signing" the document, thus verifying that the physician represents that the document is a true and correct record of the medical encounter. The document is then subjected to data extraction 190, the steps of which are shown in Fig. 6 and described in the accompanying text below.

When extraction is complete, the document may be stored 185 in a document repository 215, as shown in sub-process 210. The document repository is accessible by query 220, and the documents may be retrieved from storage 225 for editing following the steps as detailed in the preceding paragraphs.

FIG. 4 is a flow diagram showing two methods by which a document may be entered into a system other than by the methods shown in Fig. 3. Fig. 4 shows the steps of a dictation process in normal work flow, either through manual transcription or automatic voice recognition, followed by manual correction. Sub-process 290 depicts a modification to the traditional dictation by voice over a telephone. The modification is apparent in step 295, in which a guide for a desired document type is selected by the dictator (e.g., physician). The guide may provide an outline for a template of the document the dictator is prepared to produce. It should be noted, however, that templates are an optional part of the invention. The dictator may specify which guide has been selected, and thus specify which document is to be produced. The dictator may then follow the outline on the guide to dictate the correct portions, filling in the document template 300. In one embodiment, the dictator can enter basic commands, such as basic editing commands, and commands to move to the next section within the template, by pressing buttons on the telephone or by speaking command keywords.

Sub-processes 60 and 260 show the steps involved in entering dictation by use of a personal digital assistant (PDA). A template may be selected 265 from a group of templates stored in a template database 85, produced according to the method shown in Fig. 2 (82) and described above. The template may be specifically designed for use with a PDA. It should be noted, however, that templates are an optional aspect of the invention. In one embodiment, dictation into the document template may optionally be done iteratively (repeating steps 270 and 275), section by section, until the document is complete. The next incomplete section is selected in step 270, then the section is filled in by dictation 275.

Once the document has been dictated, whether by sub-process 290 (telephone dictation), or sub-process 260 (PDA dictation), the resulting document may be an audio file 280. Once the audio file 280 has been captured, it is stored in step 305 in an audio storage repository 310. A transcriptionist may retrieve 315 audio files from the repository 310, and transcribe them following steps such as those set forth in FIG. 5.

FIG. 5 is a flow diagram showing two possible routes for transcription workflow. Sub-process 320 sets forth the steps for manual transcription. The audio file may be retrieved from a persistent audio storage 310A. A transcriptionist may select the audio to be transcribed 325 and may select 330 the template 335 that is appropriate for the document type of the audio file to be transcribed. (The template may have been prepared according to the steps set forth in Fig. 2, as shown in sub-process 60, in which templates are created 82 and stored in a template database 85.) The transcriptionist listens to the audio file 280A and transcribes it within the confines of the selected template in step 340.

Sub-process 355 shows steps involved in transcription aided by automatic speech recognition (ASR). This sub-process is similar to that shown in sub-process 320, except that automatic speech recognition has been used to recreate a preliminary draft of the transcribed documents, either in a batch process, or in real time, and the transcriptionist may work from this preliminary draft rather than working with the audio file from scratch. Audio files are stored in audio storage database 310B. The audio files may be subjected to ASR 360, either via an offline batch process, or via an inline, real time process. The recognized document draft may be stored 365, or used immediately in transcription 370. A transcriptionist may select the template 390 that is appropriate for the document type of the audio file being transcribed. (The template may have been prepared according to the steps set forth in Fig. 2, as shown in sub-process 60, in which templates are created 82 and stored in a template database 85.) The transcriptionist listens to the audio file while viewing the recognized text 375, and edits and corrects the recognized text 380, while ensuring that the text is placed within the appropriate sections of the template for the current document type 385.

Once a document has been transcribed, either by sub-process 320 (direct transcription) or by sub-process 355 (ASR followed by correction), or by any other suitable process, the result is a transcribed document 350. The transcribed document may then be saved and subjected to further downstream processing, as depicted in sub-process 400. The document may be closed and saved in step 410. In step 415, data may be extracted from the transcribed document. The data extraction steps are set forth in Fig. 6, and described in detail below. The data extraction results are then stored 420 in a database document repository 430.

FIG. 6 is a flow diagram showing the steps in an embodiment of the data extraction and storage processes. A document 440, preferably conforming to a known template or document structure, may be provided for data extraction. Data extraction may begin with the identification of spans of text containing the principal medical facts, either in a manual process of recognition by a trained specialist 485, or automatically, as depicted in sub-process 445.

Sub-process 445 shows the steps in automated data extraction. Automated extraction takes into account the document type, as derived from the document's template 475. The extraction step 470 may use at least one classification engine 455 and at least one pattern matching engine 460 to apply to the text to identify spans of relevant text. Classification engines typically depend on statistical models that may be generated using training data before data extraction; the statistical models 450 may be stored for use by the classification engines. The statistical models may vary depending on document type.

The resulting product of either manual 485 or automated 445 data extraction is a set of spans of relevant text containing medical facts to be identified. In step 495, these spans of text may be manually corrected.

Sub-process 500 shows the steps involved in automatic data field extraction and normalization, in which particular low-level constituent data (i.e., particular medical facts) are extracted from the text spans containing medical facts. The data creation step 510 may use one or more parsing engines 515 to recognize the constituent data within the text spans, and create fields of data 520 containing all the constituent data within the data spans.

The data fields 520 may then be subjected to a normalization process 530. The normalization step 530 may use one or more normalization engines 525, which in turn use standard nomenclature data 540 to alter the data fields to conform to some standard, thus resulting in normalized data 535. The normalized data 535 may be subjected to manual data correction 497, to ensure that the parsing of the span of text into normalized constituent components is appropriate and correct. The normalized data may then be authenticated or digitally signed by the physician or professional who prepared the report, 549. The extracted, corrected, and normalized data may be stored 550 in a database 560, which allows database storage of the document itself in database 565, in parallel with the data extracted from the document 570.

FIG. 7 is a flow diagram showing the steps in various database queries using an embodiment of the data storage method of the invention. Persistent storage units 560 may include a structured document storage database 565 and a parallel database 570 containing the data elements extracted from the documents stored in database 565. It will be understood by those skilled in the art that the document storage does not need to be in a relational database such as database 565. Other storage vehicles or means will also suffice. Preferably, the documents in database 565 are linked to the data elements extracted therefrom, stored in database 565. Box 680 represents a data retrieval executive, which is a process embodied in a computer program to accept queries on the data and return results stored in the database.

FIG. 7 shows three distinct types of data retrieval based on query type 600. These three distinct types of data retrieval are depicted in sub-processes 710, 720, and 730. Sub-process 710 is the process by which queries for sections of previous reports may be made, where the sections of the previous reports may be specifically selected for reuse in a report that is presently being dictated. Thus, the elements of the query will be determined by the identity of the patient, and the section of the present report to be filled in. Thus, the patient demographics and the desired section from the previous report 670 are used to retrieve any relevant sections of the previous reports 675. In step 685, the dictator (e.g., physician) may select which version of the present section they wish to use. Alternatively, they may have a predetermined preference for which section to use, e.g., the corresponding section in the most recent previous report. The result is the selected section from the selected previous report 690, which is returned in step 700 to the dictation editing steps as depicted in Fig. 3 and described in the accompanying text above.

Sub-process 720 is the process by which previously recorded data is used by a physician or other medical professional at the point of care. The query field includes the patient identity 660, which is used to retrieve the relevant point of care data in step 665. The patient data to be retrieved may be customizable by site and potentially by the specialty of the provider who has requested the data, and thus there is potentially a significant amount of control over the results that are retrieved.

Sub-process 730 shows the steps involved in the use of stored data to retrieve records (e.g., medical records) that match given search criteria. This is an extremely useful feature of the invention which may be used for several purposes, including compliance, quality assurance, and quality of care audits. Step 610 decides whether the present query is new or based on a stored query. Any new or altered stored query may itself be stored 645 in a query database 580 to facilitate future queries. If a stored query is used, it is selected from the query database 580. Instantiation of a new query may involve specifying query targets 630. The query may be automatically refined 650 and then executed, and any matching records may be retrieved 655.

Matching records either from a point of care query 720 or a record retrieval query 730 may be reported or further refined. Query results can simply be reported in the user interface of a computer program 755, printed out in a report 760, or exported in various formats, e.g., text 765, HL7 770, or XML 775. Refining can involve executing another, possibly different query 780 over the results of the previous query in order to eliminate any undesirable matches. The query results may be saved 785 in a database 800. The query results may be compared with baseline results of a prior query 810 and the comparison results may be used to generate a printed report 815.

FIG. 8 is a flow diagram showing the steps in an embodiment in which previously stored documents are imported into the system for processing. A database containing previously stored documents 215 may be accessed according to workflow rules 910 specified by a customer (e.g., a particular medical facility). Documents from the database may be retrieved batch-wise 900 from the repository for processing and extraction 920(45), as detailed above in FIG. 6 and the accompanying description.

Numbered aspects of the invention include:
1. A system for managing information, the system comprising: a means for capturing text from a document source; a means for determining the structure of the captured text; a means for extracting elements of data from the captured text; a means for categorizing the extracted elements of data; a means for normalizing the extracted elements of data; a means for adding metadata relating to the captured text; a means for validating the extracted elements of data and the metadata, wherein the means for validating further includes validating at least one of the normalized extracted elements of data and the categorized extracted elements of data; a storage means for storing an electronic document including the categorized, normalized and validated extracted elements of data and metadata in a storage means, wherein the categorized, normalized and validated extracted elements of data and metadata are stored in association with the captured text; and a means for retrieving the electronic document from the storage means.
2. The system according to 1, further comprising a means for displaying the retrieved electronic records.
3. The system according to 2, further comprising a means for displaying the metadata.
4. The system according to 3, further comprising a means for displaying the captured text.
5. The system according to 1, further comprising a means for determining the structure of the format of the captured text.
6. The system according to 5, further comprising a means for determining the structure of the content of the captured text.
7. The system according to 6, wherein the content of the captured includes one of terms, words and phrases.
8. The system according to 1, further comprising a means for determining the overall classification of the content of the captured text.
9. The system according to 5, further comprising a means for reusing the extracted elements of data.
10. The system according to 5, further comprising a means for reusing the metadata.
11. The system according to 5, further comprising a means for reusing the captured text.
12. The system according to 1, further comprising a means for tracking which sections of the captured text have been completed.
13. The system according to 1, wherein the document source originates from an archive of legacy documents.
14. The system according to 13, wherein the document source includes at least one new document created from a predetermined document template.
15. The system according to 14, further comprising a means for entering text in the new document created from the predetermined document template.
16. The system according to 15, wherein the means for entering text in the new document created from a predetermined document template includes one of a keyboard attached to a computer, a microphone attached to a computer, a telephone, or a PDA.
17. The system according to 1, wherein the storage means for storing the electronic document is a computer hard drive.
18. The system according to 1, wherein the means for extracting elements of data from the text is a data extraction engine.
19. The system according to 1, wherein the means for validating extracted elements of data is a software program with access to the storage means for storing an electronic record.
20. The system according to 1, wherein the means for retrieving is accomplished by reference to at least one of the extracted elements of data, the metadata or the captured text.
21. The system according to 20, wherein the means for retrieving an electronic document from the storage means is a software program with access to the storage means for storing an electronic record.
22. The system according to 21, wherein information about the location of the extracted elements of data within the captured text is used to retrieve a section of the electronic document corresponding to the location of the extracted elements of data.
23. The system according to 22, wherein the means for adding metadata is a software program with access to the storage means for storing an electronic document.
24. The system according to 1, wherein the means for categorizing includes canonicalizing the extracted of elements of data.
25. The system according to 24, wherein the means for categorizing includes classifying headings contained in the extracted elements of data.
26. A method for managing information, the method comprising the steps of : capturing text from a document source; determining the structure of the captured text; extracting elements of data from the captured text; categorizing the extracted elements of data; normalizing the extracted elements of data; adding metadata relating to the captured text; validating the extracted elements of data and the metadata, wherein validating further includes validating at least one of the normalized extracted elements of data and the categorized extracted elements of data; storing an electronic document including the categorized, normalized and validated extracted elements of data and metadata in a storage means, wherein the categorized, normalized and validated extracted elements of data and metadata are stored in association with the captured text; and retrieving the electronic document from the storage means.
27. The method according to 26, further comprising the step of displaying the retrieved electronic records.
28. The method according to 26, further comprising the step of displaying the metadata.
29. The method according to 28, further comprising the, step of displaying the captured text.
30. The method according to 26, further comprising the step of determining the structure of the format of the captured text.
31. The method according to 30, further comprising the step of determining the structure of the content of the captured text.
32. The method according to 31, wherein the content of the captured includes one of terms, words and phrases.
33. The method according to 26, further comprising the step of determining the overall classification of the content of the captured text.
34. The method according to 30, further comprising the step of reusing the extracted elements of data.
35. The method according to 30, further comprising the step of reusing the metadata.
36. The method according to 30, further comprising the step of reusing the captured text.
37. The method according to 26, further comprising the step of tracking which sections of the captured text have been completed.
38. The method according to 26, wherein the document source originates from an archive of legacy documents.
39. The method according to 38, wherein the document source includes at least one new document created from a predetermined document template.
40. The method according to 39, further comprising the step of entering text in the new document created from the predetermined document template.
41. The method according to 40, wherein step of entering text in the new document created from a predetermined document template includes one of a keyboard attached to a computer, a microphone attached to a computer, a telephone, or a PDA.
42. The method according to 41, wherein the step of storing the electronic document is storing the electronic document on a computer hard drive.
43. The method according to 26, wherein the step of extracting elements of data from the text includes using a data extraction engine.
44. The method according to 26, wherein the step of validating extracted elements of data includes using a software program with access to the storage means for storing an electronic record.
45. The method according to 26, wherein the step of retrieving is accomplished by reference to at least one of the extracted elements of data, the metadata or the captured text.
46. The method according to 45, wherein the step of retrieving an electronic document includes using is a software program with access to the storage means for storing an electronic record.
47. The method according to 46, wherein information about the location of the extracted elements of data within the captured text is used to retrieve a section of the electronic document corresponding to the location of the extracted elements of data.
48. The method according to 47, wherein the step of adding metadata includes using a software program with access to the storage means.
49. The method according to 26, wherein step of categorizing includes canonicalizing the extracted of elements of data.
50. The method according to 49, wherein the step of categorizing includes classifying headings contained in the extracted elements of data.
51. A method, the method comprising the steps of : entering information text in a predetermined record template, wherein text pertinent to a section of the template is entered in that section as natural language; storing a completed medical record; extracting elements of medical data from the text, wherein information about which section of the template the text was entered is used to extract elements of medical data from the text; validating extracted data; normalizing the extracted elements of medical data; storing an electronic medical record as normalized extracted elements of medical data in association with the text from which they were extracted; and retrieving electronic medical records from the storage means by natural language searching, wherein the retrieving is accomplished by reference to one or more extracted elements and the natural language is normalized.
52. The method according to 51, wherein the step of entering medical information text is performed using a microphone attached to a computer.
53. The method according to 51, wherein the step of entering medical information is performed using a telephone.
54. The method according to 51, wherein the step of entering medical information is performed using a PDA.
55. The method according to 51, wherein the step of storing a completed medical record and the step of storing an electronic medical record are performed on computer hard drives.
56. The method according to 55, wherein the step of storing a completed medical record and step of storing an electronic medical record are performed on database servers.
57. The method according to 51, wherein the step of extracting elements of medical data from the text is performed using a NLP and ML data extraction engine.
58. The method according to 51, wherein the step of validating extracted data is performed using a web browser software program with access to the stored electronic medical record.
59. The method according to 51, wherein the step of normalizing the extracted elements of medical data is performed using a software program running on a computer.
60. The method according to 51, wherein the step of retrieving electronic medical records is performed using a web browser software program with access to the stored electronic medical record.
61. The method according to 51, further comprising the step of creating a medical record template.
62. The method according to 51, further comprising providing a patient history summary.
63. The method according to 51, further comprising the step of adding medical metadata.
64. The method according to 63, wherein the additional medical metadata is stored on a computer hard drive.
65. The method according to 63, wherein the additional medical metadata is stored on a database server.
66. The method according to 63, wherein the step of adding medical metadata is performed using a software program running on a computer.
67. The method according to 63, wherein the step of adding medical metadata is performed using a web browser with access to the stored electronic medical record.

## Claims

1. A method comprising:
extracting elements of medical data from text in medical reports using natural language processing, wherein the extracting comprises normalizing to a standard set of medical terms;
storing the extracted elements in a database in association with the text from which they were extracted;
normalizing a search query to the standard set of medical terms; and
utilizing the normalized search query to search the extracted elements in the database to retrieve associated medical reports matching the search query.

2. The method of claim 1, wherein the text to which the natural language processing is applied comprises a free-form dictation provided by a medical professional.

3. The method of claim 1 or 2, wherein the extracted elements of medical data represent one or more items of information selected from the group consisting of patient complaints, symptoms, diagnoses, treatments, procedures, allergies, medicines, laboratory test results, vaccinations, and immunizations.

4. The method of any preceding claim, wherein the standard set of medical terms comprises classification codes.

5. The method of any preceding claim, wherein the standard set of medical terms conforms to the International Classification of Diseases (ICD) standard, the Current Procedural Terminology (CPT) standard, and/or the Systematized Nomenclature of Medicine (SNOMED) standard.

6. The method of any preceding claim, further comprising presenting the extracted elements of medical data to a medical professional or clinical data specialist for verification.

7. The method of any preceding claim, wherein the text in a medical report is organized in sections, and wherein the extracting comprises using information about which section the text is in to extract the elements of medical data from the text.

8. The method of claim 7, wherein the storing comprises storing the text and extracted elements in association with the section the text is from.

9. The method of claim 8, wherein the searching is performed section by section.

10. The method of any preceding claim, wherein retrieving the associated medical reports comprises retrieving medical records of patients with a particular ailment, undergoing a particular treatment, and/or taking a particular medication.

11. The method of any of claims 1-9, wherein retrieving the associated medical reports comprises retrieving instances within an individual patient's record of one or more ailments, treatments, and/or medications.

12. The method of any preceding claim, further comprising storing the search query in a query database to facilitate future queries.

13. The method of any preceding claim, further comprising using the extracted elements of medical data to maintain a list of current allergies, problems, and/or medications for a patient.

14. A computer program product comprising program code means which, when executed by at least one computer, perform the method of any preceding claim.

15. Apparatus comprising at least one processor programmed to perform the method or execute the computer program of any preceding claim.
